# EUROPEAN PATENT APPLICATION

(11) **EP 1 978 035 A1**
(43) Date of publication of application: **08.10.2008**
(21) Application number: 07090073.3
(22) Date of filing: 05.04.2007
(51) Int. Cl.: C07K 16/18, A61K 39/395, G01N 33/563, C12N 15/13

(54) **Anti-amyloid antibodies and their use in diagnosis and therapy of amyloid diseases**

(71) Applicant: Hans-Knöll-Institut Leibniz-Institut für Naturstoff-Forschung, 07745 Jena (DE); Fritz-Lipmann-Institut e.V. Leibniz-Institut für Altersforschung, 07745 Jena (DE)
(72) Inventor: Horn, Uwe, 06567 Rottleben (DE); Hortschansky, Peter, 07768 Kahla (DE); Habicht, Gernot, 04545 Gera (DE); Fändrich, Marcus, 07743 Jena (DE)
(74) Representative: Boeckh, Tobias

(57) **Abstract**

The invention concerns antibodies to amyloid fibrillar and non-fibrillar polypeptides. The invention further concerns the use of such antibodies in diagnosis, treatment and/or prevention of amyloid diseases. The present invention also provides preparations and methods for preventing and treating, in a mammal, a disease characterized by amyloid formation and/or aggregation, preferably by promoting a non-fibrillar aggregation.

## Description

The invention concerns antibodies to amyloid fibrillar and non-fibrillar polypeptides. The invention further concerns the use of such antibodies in diagnosis, treatment and/or prevention of amyloid diseases. The present invention also provides preparations and methods for preventing and treating, in a mammal, a disease characterized by amyloid formation and/or aggregation, preferably by promoting a non-fibrillar aggregation.

Amyloid diseases or amyloidosis refers to a pathological condition in a mammal characterized by the presence of amyloid fibers. Amyloid is a generic term referring to a group of diverse but specific protein deposits. All amyloid deposits have common morphologic properties, stain with specific dyes (e.g. Congo red), and have a characteristic red-green birefringent appearance in polarized light after staining. Different amyloids are also characterized by the type of protein present in the deposit. For example, neurodegenerative diseases such as scrapie, bovine spongiform encephalitis, Creutzfeldt-Jakob disease and the like are characterized by the appearance and accumulation of a protease-resistant form of prion protein in the nervous system. Similarly, Alzheimer's disease, another neurodegenerative disorder, is characterized by neuritic plaques and neurofibrillary tangles. In this case, the plaque and blood vessel amyloid is formed by the deposition of fibrillar beta-amyloid protein.

Amyloid material deposition (also referred to as amyloid plaque formation) is also a central feature of a variety of unrelated pathological conditions including Alzheimer's disease, prion-related encephalopathies, type II diabetes mellitus, familial amyloidosis and light-chain amyloidosis.

Amyloid material is composed of a dense network of rigid, nonbranching proteinaceous fibrils of indefinite length that are about 80 to 100 A in diameter. Amyloid fibrils contain a core structure of polypeptide chains arranged in antiparallel or parallel beta-pleated sheets lying with their long axes perpendicular to the long axis of the fibril.

Approximately twenty amyloid fibril proteins have been identified in-vivo and correlated with specific diseases. These proteins share little or no amino acid sequence homology, however the core structure of the amyloid fibrils is essentially the same. This common core structure of amyloid fibrils and the presence of common substances in amyloid deposits suggest that data characterizing a particular form of amyloid material may also be relevant to other forms of amyloid material and thus can be implemented in template design for the development of drugs against amyloid-associated diseases such as type II diabetes mellitus, Alzheimer's dementia or diseases and prion-related encephalopathies.

Furthermore, amyloid deposits do not appear to be inert in vivo, but rather are in a dynamic state of turnover and can even regress if the formation of fibrils is halted.

Thus, therapies designed to inhibiting amyloidosis may be useful for treating amyloid associated diseases.

Inhibition or modification of the formation of amyloid fibrils--Amyloid, including islet amyloid, contains potential stabilizing or protective substances, such as serum amyloid P component, apolipoprotein E, and perlecan. Blocking their binding to developing amyloid fibrils could inhibit amyloidogenesis, as could treatment with antibodies specific for certain parts of an amyloidogenic protein.

Accordingly, there exists the need for new therapies and reagents for the treatment of amyloid disease, in particular, therapies and reagents capable of effecting a therapeutic benefit at physiologic doses.

The present invention features new immunological reagents, in particular, therapeutic peptides/binding peptides or antibody reagents for the prevention and treatment of amyloidogenic disease. The invention is based, at least in part, on the identification and characterization of antibodies that specifically bind to amyloid fibrillar/non-fibrillar polypeptides and are effective at reducing plaque burden and/or reducing the neuritic dystrophy associated with amyloidogenic disorders. Structural and functional analysis of these antibodies leads to the design of various humanized antibodies for prophylactic and/or therapeutic use. In particular, the invention features humanization of the variable regions of these antibodies and, accordingly provides for humanized immunoglobulin or antibody chains, intact humanized immunoglobulins or antibodies, and functional immunoglobulin or antibody fragments, in particular, antigen binding fragments, of the featured antibodies. The invention also provides methods for treating or preventing diseases associated with amyloid/beta-amyloid deposit in an individual, such as Alzheimer's disease, Down's syndrome, multi-infarct dementia, mild cognitive impairment, cerebral amyloid angiopathy, depression, Creutzfeldt-Jakob disease, and dementia with Lewy body in a subject by administering to the subject an effective amount of a pharmaceutical composition comprising an antibody, a polypeptide, or a polynucleotide encoding the antibody or the polypeptide described herein.

Also featured are antibodies (e.g., humanized antibodies) having altered effector functions, and therapeutic uses thereof.

Surprisingly, the peptide of the invention, characterized in that it is selected from the group comprising:
a) a peptide consisting of the amino acid sequence according to SEQ ID No. 1, preferably SEQ ID No. 2, 3 and/or 4.
b) a peptide consisting of an amino acid sequence having sufficient homology to be functionally analogous/equivalent to an amino acid sequence in accordance with a);
c) a peptide according to an amino acid sequence a) or b) which is modified by deletions, additions, substitutions, translocations, inversions and/or insertions and functionally analogous/equivalent to an amino acid sequence in accordance with a) or b);
d) a peptide according to amino acid sequence a), b) or c) which is modified by branch or extension with the same or another peptide according to amino acid sequence a), b) or c) to form a homooligomeric or heterooligomeric peptide,
interacts in a specific manner with amyloid fibrillar/non-fibrillar polypeptides.

In the meaning of the invention the term "to be functionally analogous" or "equivalent" to the above-mentioned sequences means that the homologues exhibit a behavior in amyloid diseases such that reliable and effective use thereof is possible in diagnosis and/or therapy of such diseases or pathogenic conditions associated with said diseases. Functionally analogous sequences in the meaning of the invention are all those sequences which can be identified as equally effective by a person skilled in the art, using routine tests.

Even if the substance of the invention consists of the amino acid sequences SEQ ID No. 1, 2, 3 and/or 4, wherein SEQ ID Nos. 2, 3 and 4 are preferably binding domains, it is known to those skilled in the art that the substance of the invention may be modified by addition, deletion or substitution without essentially altering the polypeptide. The modified amino acid sequence is not essentially altered as long as it fulfils the same function as the sequences SEQ ID No. 1, 2, 3 and/or 4 in essentially the same way, leading to essentially the same outcome. In the meaning of the invention, the modified amino acid sequence thus essentially corresponds particularly to the amino acid sequences SEQ ID No. 1, 2, 3 and/or 4, if applying it does not essentially influence the solution of the problem of the invention and if this is obvious for a person skilled in the art, who realises that the inventors do not intend to limit their teaching to the wording of the claims - that is the sequences according to SEQ ID No. 1, 2, 3 and/or 4 or its modifications.

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney, ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-1998) J. Wiley and Sons; Methods in Enzymology (Academic Press, Inc.); Handbook of Experimental Immunology (D. M. Weir and C. C. Blackwell, eds.); Gene Transfer Vectors for Mammalian Cells (J. M. Miller and M. P. Calos, eds., 1987); Current Protocols in Molecular Biology (F. M. Ausubel et al., eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: a practical approach (D. Catty., ed., IRL Press, 1988-1989); Monoclonal antibodies: a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J. D. Capra, eds., Harwood Academic Publishers, 1995).

In certain embodiments, the present invention concerns novel compositions comprising at least one protein or peptide. As used herein, a protein or peptide generally refers, but is not limited to, a protein of greater than about 200 amino acid; a polypeptide of greater than about 100 amino acids; and/or a peptide of from about 3 to about 100 amino acids. For convenience, the terms "polypeptide", "oligopeptide", "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. It is understood that, because the polypeptides of this invention are based upon an antibody, the polypeptides can occur as single chains or associated chains.

In certain embodiments the size of the protein or peptide may comprise, but is not limited to, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, about 110, about 120, about 130, or greater amino acid residues.

As used herein, an "amino acid residue" refers to any naturally occurring amino acid, any amino acid derivative or any amino acid mimic known in the art. In certain embodiments, the residues of the protein or peptide are sequential, without any non-amino acid interrupting the sequence of amino acid residues. In other embodiments, the sequence may comprise one or more non-amino acid moieties. In particular embodiments, the sequence of residues of the protein or peptide may be interrupted by one or more non-amino acid moieties.

Accordingly, the term "protein or peptide" encompasses amino acid sequences comprising at least one of the 20 common amino acids found in naturally occurring proteins, or at least one modified or unusual amino acid, 2-Aminoadipic acid, N-Ethylasparagine, 3-Aminoadipic acid, Hydroxylysine, beta-alanine, beta-Amino-propionic acid, allo-Hydroxylysine, 2-Aminobutyric acid, 3-Hydroxyproline, 4-Aminobutyric acid, piperidinic, 4-Hydroxyproline acid, 6-Aminocaproic acid, Isodesmosine, 2-Aminoheptanoic acid, allo-Isoleucine, 2-Aminoisobutyric acid, N-Methylglycine, sarcosine, 3-Aminoisobutyric acid, N-Methylisoleucine, 2-Aminopimelic acid, 6-N-Methyllysine, 2,4-Diaminobutyric acid, N-Methylvaline, Desmosine, Norvaline, 2,2'-Diaminopimelic acid, Norleucine, 2,3-Diaminopropionic acid, Ornithine and/or N-Ethylglycine.

Proteins or peptides may be made by any technique known to those of skill in the art, including the expression of proteins, polypeptides or peptides through standard molecular biological techniques, or the chemical synthesis of proteins or peptides. Alternatively, various commercial preparations of proteins, polypeptides and peptides are known to those of skill in the art and proteins or peptides of specific sequence may be obtained from a variety of commercial vendors known in the art (e.g., Midland Certified Reagents, Midland, Tex.). Peptides containing derivatized amino acid residues may be prepared by incorporating such residues into the peptide chain during synthesis. Alternatively, modified amino acid residues may be prepared by chemical derivatization after peptide synthesis, for example using side-chain specific chemical modifying agents well known in the art.

Another embodiment for the preparation of polypeptides according to the invention is the use of peptide mimetics. Mimetics are peptide-containing molecules that mimic elements of protein secondary structure. See, for example, Johnson et al., "Peptide Turn Mimetics" in BIOTECHNOLOGY AND PHARMACY, Pezzuto et al., Eds., Chapman and Hall, New York (1993). The underlying rationale behind the use of peptide mimetics is that the peptide backbone of proteins exists chiefly to orient amino acid side chains in such a way as to facilitate molecular interactions, such as those of antibody and antigen. A peptide mimetic is expected to permit molecular interactions similar to the natural molecule. These principles may be used to engineer second generation molecules having many of the natural properties of the peptides of the invention (= binding peptides) disclosed herein, but with altered and even improved characteristics.

In certain embodiments, the present invention may concern fusion proteins. These molecules may have all or a substantial portion of a binding peptide of the invention, linked at the N- or C-terminus, to all or a portion of a second polypeptide or protein. For example, fusions may employ leader sequences from other species to permit the recombinant expression of a protein in a heterologous host. Inclusion of a cleavage site at or near the fusion junction may be used to facilitate removal of the extraneous polypeptide after purification. In other cases, a leader sequence may be included to facilitate intracellular targeting of the binding peptide of the invention. Other useful fusions may include linking of functional domains, such as active sites from enzymes or transmembrane regions. These examples are not meant to be limiting and it is contemplated that within the scope of the present invention virtually any protein or peptide could be incorporated into a fusion protein comprising the binding peptide of the invention. Methods of generating fusion proteins are well known to those of skill in the art. Such proteins can be produced, for example, by chemical attachment using bifunctional cross-linking reagents, by de novo synthesis of the complete fusion protein, or by attachment of a DNA sequence encoding the anti-amyloid binding peptide to a DNA sequence encoding the second peptide or protein, followed by expression of the intact fusion protein.

Certain embodiments may concern isolation and/or purification of the anti-amyloid binding peptide of the invention. Protein purification techniques are well known to those of skill in the art. The protein or peptide of interest may be purified using chromatographic and electrophoretic techniques to achieve partial or complete purification (or purification to homogeneity). Analytical methods particularly suited to the preparation of a pure peptide are ion-exchange chromatography, gel exclusion chromatography, polyacrylamide gel electrophoresis, affinity chromatography, immunoaffinity chromatography and isoelectric focusing. A particularly efficient method of purifying peptides is fast protein liquid chromatography (FPLC) or even HPLC. For short peptides, reverse-phase HPLC may be of use.

Various techniques suitable for use in protein purification are well known to those of skill in the art. These include, for example, precipitation with ammonium sulphate, PEG, antibodies and the like, or by heat denaturation, followed by: centrifugation; chromatography steps such as ion exchange, gel filtration, reverse phase, hydroxylapatite and affinity chromatography; isoelectric focusing; gel electrophoresis; and combinations of these and other techniques. As is generally known in the art, it is believed that the order of conducting the various purification steps may be changed, or that certain steps may be omitted, and still result in a suitable method for the preparation of a substantially purified protein or peptide.

There is no general requirement that the protein or peptide always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed utilizing an HPLC apparatus will generally result in a greater "-fold" purification than the same technique utilizing a low pressure chromatography system. Methods exhibiting a lower degree of relative purification may have advantages in total recovery of protein product, or in maintaining the activity of an expressed protein.

Affinity chromatography is a chromatographic procedure that relies on the specific affinity between a substance to be isolated and a molecule to which it can specifically bind to. This is a receptor-ligand type of interaction. The column material is synthesized by covalently coupling one of the binding partners to an insoluble matrix. The column material is then able to specifically adsorb the substance from the solution. Elution occurs by changing the conditions to those in which binding will not occur (e.g., altered pH, ionic strength, temperature, etc.). The matrix should be a substance that itself does not adsorb molecules to any significant extent and that has a broad range of chemical, physical and thermal stability. The ligand should be coupled in such a way as to not affect its binding properties. The ligand should also provide relatively tight binding. And it should be possible to elute the substance without destroying the sample or the ligand.

In a preferred embodiment, the invention relates to a peptide wherein the amino acid sequence specified under b) has at least 40% homology to any of the amino acid sequences specified under a).

In another preferred embodiment, the invention relates to a peptide wherein the amino acid sequence specified under b) has at least 60%, preferably 70%, more preferably 80%, especially preferably 90% homology to any of the amino acid sequences specified under a).

In another preferred embodiment, the invention relates to a peptide which consists of the amino acid sequence SEQ ID No. 1.

In a preferred embodiment, the invention relates to a peptide which comprises or preferably consists of the amino acid sequence SEQ ID No. 2.

In another preferred embodiment, the invention relates to a peptide which comprises or preferably consists of the amino acid sequence SEQ ID No. 3.

In another preferred embodiment, the invention relates to a peptide which comprises or preferably consists of the amino acid sequence SEQ ID No. 4. In a more preferred embodiment, the sequences SEQ ID No. 2, 3 and/or 4 are binder to the amyloid.

In another preferred embodiment, the invention relates to a peptide comprising an amino acid sequence wherein the linker and/or a spacer are selected from the group comprising: α-aminocarboxylic acids as well as homo- and heterooligomers thereof, α,ω-aminocarboxylic acids and branched homo- or heterooligomers thereof, other aliphatic and/or aromatic amino acids as well as linear and branched homo- or heterooligomers; amino-oligoalkoxyalkylamines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkylmercaptophenoxy derivatives; 4-oligo-alkylaminophenyl or 4-oligoalkylaminophenoxy derivatives; (oligoalkylbenzyl)phenyl or 4-(oligoalkylbenzyl)phenoxy derivatives, as well as 4-(oligo-alkoxybenzyl)phenyl or 4-(oligoalkoxy-benzyl)phenoxy derivatives; trityl derivatives; benzyloxyaryl or benzyloxyalkyl derivatives; xanthen-3-yloxyalkyl derivatives; (4-alkylphenyl)- or m-(4-alkylphenoxy)alkanoic acid derivatives; oligoalkyl-phenoxyalkyl or oligoalkoxyphenoxyalkyl derivatives; carbamate derivatives; amines; trialkylsilyl or dialkylalkoxysilyl derivatives; alkyl or aryl derivatives and/or combinations thereof.

In another preferred embodiment, the invention relates to a peptide comprising an amino acid sequence X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36-X37-X38-X39-X40-X41-X42-X43-X44-X45-X46-X47-X48-X49-X50-X51-X52-X53-X54-X55-X56-X57-X58-X59-X60-X61-X62-X63-X64-X65-X66-X67-X68-X69-X70-X71-X72-X73-X74-X75-X76-X77-X78-X79-X80-X81-X82-X83-X84-X85-X86-X87-X88-X89-X90-X-1-X92-X93-X94-X95-X96-X97-X98-X99-X100-X101-X102-X103-X104-X105-X106-X107-X108-X109-X110-X111-X112-X113-X114-X115-X116-X117-X118-X119-X120-X121-X122-X123-X124-X125-X126-X127-X128-X129-X130-X131 wherein

| | |
|---|---|
| X1 = | amino group, amide, acetyl group, biotin group, marker, spacer, linker, GKK, SGKK or deletion, |
| X2 = | E* |
| X3 = | V# |
| X4 = | Q# |
| X5 = | L# |
| X6 = | V# |
| X7 = | E# |
| X8 = | S# |
| X9 = | G# |
| X10 = | G# |
| X11 = | G# |
| X12 = | L# |
| X13 = | V# |
| X14 = | Q# |
| X15 = | P* |
| X16 = | G* |
| X17 = | G# |
| X18 = | S# |
| X19 = | L# |
| X20 = | R# |
| X21 = | L# |
| X22 = | S# |
| X23 = | C# |
| X24 = | T# |
| X25 = | A# |
| X26 = | S# |
| X27 = | G# |
| X28 = | Y* |
| X29 = | T* |
| X30 = | F* |
| X31 = | S* |
| X32 = | H#, R#, K# |
| X33 = | R#, H#, K# |
| X34 = | Y#, W#, F#, I#, L#, V#, T#, S# |
| X35 = | H#, R#, K# |
| X36 = | R#, H#, K# |
| X37 = | W# |
| X38 = | F# |
| X39 = | R# |
| X40 = | Q# |
| X41 = | A# |
| X42 = | P* |
| X43 = | G* |
| X44 = | K* |
| X45 = | E# |
| X46 = | R# |
| X47 = | E# |
| X48 = | I# |
| X49 = | V# |
| X50 = | A# |
| X51 = | V# |
| X52 = | I# |
| X53 = | S#, T#, W#, V#, I#, L# |
| X54 = | Q, N |
| X55 = | S, T |
| X56 = | G, A |
| X57 = | M#, C#, V#, I#, L#, Y#, W#, F# |
| X58 = | R#, K#, H# |
| X59 = | T# |
| X60 = | Y# |
| X61 = | Y# |
| X62 = | A# |
| X63 = | D* |
| X64 = | S* |
| X65 = | V* |
| X66 = | K* |
| X67 = | G* |
| X68 = | R# |
| X69 = | F# |
| X70 = | T# |
| X71 = | I# |
| X72 = | S# |
| X73 = | R# |
| X74 = | D# |
| X75 = | N* |
| X76 = | A* |
| X77 = | K* |
| X78= | N* |
| X79 = | T# |
| X80 = | V# |
| X81 = | Y# |
| X82 = | L# |
| X83 = | Q# |
| X84 = | M# |
| X85 = | N# |
| X86 = | S* |
| X87 = | L* |
| X88 = | K*, R*, S*, T*, A*, C*, D*, E*, F*, G*, H*, I*, L*, M*, N*, P*, Q*, V*, W*, Y* |
| X89 = | P* |
| X90 = | E* |
| X91 = | D* |
| X92 = | T#, I#, Y#, F#, V#, C#, M#, W#, Q#, E#, S#, K#, R#, H#, L# |
| X93 = | A# |
| X94 = | M# |
| X95 = | Y# |
| X96= | Y# |
| X97 = | C# |
| X98 = | A# |
| X99 = | A# |
| X100 = | G#, A# |
| X101 = | T#, S#, F#, W#, I#, L#, V# |
| X102 = | R, K, H |
| X103 = | K, H, R |
| X104 = | N, Q |
| X105 = | V, I, L, M |
| X106 = | W, F, Y |
| X107 = | T, S |
| X108 = | R, K, H |
| X109 = | Q, N |
| X110 = | H, K, R |
| X111 = | P* |
| X112 = | F* |
| X113 = | D# |
| X114 = | Y# |
| X115 = | W# |
| X116 = | G# |
| X117 = | Q# |
| X118 = | G# |
| X119 = | T# |
| X120 = | Q# |
| X121 = | V# |
| X122 = | T# |
| X123 = | V# |
| X124 = | S# |
| X125 = | S* |
| X126 = | A* |
| X127 = | S* |
| X128 = | G* |
| X129 = | A* |
| X130 = | E* |
| X131 = | amino group, amide, acetyl group, biotin group, marker, spacer, linker, GKK, SGKK or deletion, wherein |

in a preferred embodiment, the CDR-regions are underlined. Amino acids, which are marked with a star (*), are variable. In some preferred embodiments, the amino acids marked with a star are modified to any one of amino acids A, C, D, E, F, G, H, I, K, L, M, N, P, Q, R, S, T, V, W, Y. In another preferred embodiment, the amino acids marked with a star, can be deleted. In one aspect, the invention relates to an antibody which comprises a peptide with the general amino acid sequence X1 to X131, wherein the amino acid according to (i) about X1 to X31, (ii) about X37 to X52, (iii) about X59 to X99 and (iv) about X111 to X131 are substantially identical or homologue to the above-mentioned sequences (i) to (iv) and unrestrictedly variable in the part of about X32 to X36, about X53 to X58 and about X100 to X110. Amino acids, which are marked by a hash sign (#) are beta-strands; in a preferred embodiment, the introduction of singular beta-sheet breakers may not have any influence. At least 15%, 20%, 25%, 30%, 35%, 40%, or 50% of the amino acids which are not underlined can be exchanged by any type of natural and/or artificial amino acid without influencing the biological activity according to the invention, that is the interaction with amyloid fibrillar/non fibrillar polypeptides.

In another preferred embodiment, the invention relates to a peptide which is used as a medical active substance.

In another preferred embodiment, the invention relates to a peptide which is bound by amyloid fibrillar and/or non-fibrillar polypeptides of patients suffering from amyloid disease.

In a preferred embodiment, the peptide binds amyloid with much higher affinity than Congo red. The peptide is a conformation-sensitive amyloid binder, which interacts with pathogenic aggregates, soluble oligomeres or proto-fibrils.

In another preferred embodiment, the invention relates to a peptide which is immobilized and/or fixed to magnetic, paramagnetic and/or non magnetic nanoparticles.

In certain embodiments of the invention, one or more labels may be attached to the peptide of the invention. A number of different labels may be used, such as fluoro-phores, chromophores, radioisotopes, enzymatic tags, antibodies, bioluminescent, electroluminescent, phosphorescent, affinity labels, nanoparticles, metal nanoparticles, gold nanoparticles, silver nanoparticles, magnetic particles, spin labels or any other type of label known in the art.

Non-limiting examples of affinity labels include an antibody, an antibody fragment, a receptor protein, a hormone, biotin, DNP, and any polypeptide/protein molecule that binds to an affinity label.

Non-limiting examples of enzymatic tags include urease, alkaline phosphatase or peroxidase. Colorimetric indicator substrates can be employed with such enzymes to provide a detection means visible to the human eye or spectrophotometrically.

Non-limiting examples of photodetectable labels include Alexa 350, Alexa 430, AMCA, aminoacridine, BODIPY 630/650, BODIPY 650/665, BODIPY-FL, BODIPY-R6G, BODIPY-TMR, BODIPY-TRX, 5-carboxy-4',5'-dichloro-2- ',7'-dimethoxy fluorescein, 5-carboxy-2',4',5',7'-tetrachlorofluorescein, 5-carboxyfluorescein, 5-carboxyrhodamine, 6-carboxyrhodamine, 6-carboxytetramethyl amino, Cascade Blue, Cy2, Cy3, Cy5,6-FAM, dansyl chloride, Fluorescein, HEX, 6-JOE, NBD (7-nitrobenz-2-oxa-1,3-diazole), Oregon Green 488, Oregon Green 500, Oregon Green 514, Pacific Blue, phthalic acid, terephthalic acid, isophthalic acid, cresyl fast violet, cresyl blue violet, brilliant cresyl blue, para-aminobenzoic acid, erythrosine, phthalocyanines, azome-thines, cyanines, xanthines, succinylfluoresceins, rare earth metal cryptates, europium trisbipyridine diamine, a europium cryptate or chelate, diamine, dicyanins, La Jolla blue dye, allopycocyanin, allococyanin B, phycocyanin C, phycocyanin R, thiamine, phycoerythrocyanin, phyco-erythrin R, REG, Rhodamine Green, rhodamine isothiocyanate, Rhodamine Red, ROX, TAMRA, TET, TRIT (tetramethyl rhodamine isothiol), Tetramethylrhodamine, and Texas Red. These and other luminescent labels may be obtained from commercial sources such as Molecular Probes (Eugene, O-reg.).

In other embodiments of the invention, labels of use may comprise metal nanoparticles. Methods of preparing nanoparticles are known. Nanoparticles may also be obtained from commercial sources. Modified nanoparticles are available commercially, such as Nanogold.RTM. nanoparticles from Nanoprobes, Inc. (Yaphank, N.Y.).

In some embodiments of the invention, proteins may be labeled using side-chain specific and/or selective reagents. Such reagents and methods are known in the art. Non-limiting exemplary reagents that may be used include acetic anhydride (lysine, cysteine, serine and tyrosine); trinitrobenzenesulfonate (lysine); carbodiimides (glutamate, aspartate); phenylglyoxal (arginine); 2,3-butanedione (arginine); pyridoxal phosphate (lysine); p-chloromercuribenzoate (cysteine); 5,5'-dithiobis(2-nitro-benz- oic acid) (cysteine); diethylpyrocarbonate (lysine, histidine); N-bromosuccinimide (tryptophan) and tetrani-tromethane (cysteine, tyrosine). Various methods for attaching labels to nucleic acids and/or oligonucleotides are known in the art and may be used. For example, watersoluble carbodiimides may be used to cross-link the phosphate groups of nucleic acids or oligonucleotides to various labels. Amino or sulfhydryl modified oligonucleotides or nucleic acids may be attached to labels using known bifunctional crosslinking reagents.

In alternative embodiments of the invention, various cross-linking reagents known in the art, such as homo-bifunctional, hetero-bifunctional and/or photoactivatable cross-linking reagents may be used. Non-limiting examples of such reagents include bisimidates; 1,5-difluoro-2,4-(dinitrob- enzene); N-hydroxysuccinimide ester of suberic acid; disuccinimidyl tartarate; dimethyl-3,3'-dithio-bispropionimidate; N-succinimidyl-3-(2-pyridyldithio)propionate; 4-(bromoaminoethyl)-2-nitro-phenylazide; and 4-azidoglyoxal. Such reagents may be modified to attach various types of labels, such as fluorescent labels. The skilled artisan will realize that such cross-linking reagents are not limited to use with proteins, but may also be used with other types of molecules.

In another preferred embodiment, the invention relates to a peptide which is bound to a solid phase.

The invention also relates to an isolated nucleic acid molecule selected from the group comprising
a) a nucleic acid molecule comprising a nucleotide sequence which encodes at least one peptide of the invention, particularly according to SEQ ID No. 1 and/or SEQ ID No. 2, 3 and/or 4.;
b) a nucleic acid molecule which is complementary to a nucleotide sequence in accordance with a);
c) a nucleic acid molecule which undergoes hybridization with a nucleotide sequence according to a) or b) under moderately or preferred stringent conditions;
d) a nucleic acid molecule comprising a nucleotide sequence having sufficient homology to be functionally analogous/equivalent to a nucleotide sequence according to a), b) or c);
e) a nucleic acid molecule which, as a consequence of the genetic code, is degenerated into a nucleotide sequence according to a) through d); and
f) a nucleic acid molecule according to a nucleotide sequence of a) through e) which is modified by deletions, additions, substitutions, translocations, inversions and/or insertions and functionally analogous/equivalent to a nucleotide sequence according to a) through e).

The present invention also relates to a nucleic acid preparation comprising a nucleic acid sequence capable of encoding the peptide/peptide variant of the invention a fragment thereof. The nucleic acids of the invention can be DNA or RNA. A DNA molecule is "capable of expressing" a polypeptide, such as the antibody of the invention or the N-terminal fragment thereof or the C-terminal fragment thereof, if it contains nucleotide sequences which contain transcriptional and translational regulatory information, and such sequences are operably linked to nucleotide sequences which encode the peptide/polypeptide. An operable linkage is a linkage in which the regulatory DNA sequences and the DNA sequence sought to be expressed are connected in such a way as to permit gene expression. The regulatory regions needed for gene expression, in general, include a promoter region as well as the DNA sequences which, when transcribed into RNA, will signal the initiation of protein synthesis. Such regions will normally include those 5'-non-coding sequences involved in initiation of transcription and translation. A promoter region would be operably linked to a DNA sequence if the promoter were capable of effecting transcription of that DNA sequence. The nucleic acid segment encoding the peptide or peptide variant of the invention or the N-terminal fragment thereof or the C-terminal fragment thereof should thus be linked to regulatory elements, such as a promoter and enhancer, that allow expression of the nucleic acid segment in the intended host. For expression in blood cells, for example, as is desirable for induction of an immune response in a mammal, promoter and enhancer elements from light or heavy chain immunoglobulin genes or the CMV major intermediate early promoter and enhancer are suitable to direct expression.

The linked regulatory elements and coding sequences are often cloned into a vector. A vector is a nucleic acid, preferably a DNA molecule, capable of autonomous replication in a cell, to which a nucleic acid segment (e. g. a gene or polynucleotide, preferably DNA) can be operatively linked so as to bring about replication of the attached segment. Vectors capable of directing the expression of nucleic acid (preferably DNA) segments (genes) encoding for one or more proteins are referred to as "expression vectors". An expression vector thus is any plasmid or virus into which a foreign nucleotide sequence (preferably DNA) may be inserted or expressed. A number of viral vector systems are available, including (but not limited to) retroviral systems (Lawrie and Tumin, 1993), adenoviral vectors (Bett et al., 1993), adeno-associated virus vectors (Zhou et al., 1994), viral vectors from the pox family including vaccinia virus and the avian pox viruses, viral vectors from the alpha virus genus such as those derived from Sindbis and Semliki Forest Viruses (Dubensky et al., 1996), and papillomaviruses (Ohe et al., 1995; WO 94/12629; Xiao and Brandsma, 1996).

As used herein, "stringent conditions" or "high stringency conditions" are those that: (1) employ low ionic strength and high temperature for washing, for example 0.015 M sodium chloride/0.0015 M sodium citrate/0.1% sodium dodecyl sulfate at 50 degree C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42 degree C.; or (3) employ 50% formamide, 5 times SSC (0.75 M NaCl, 0.075 M sodium citrate), 50 mM sodium phosphate (pH 6.8), 0.1% sodium pyrophosphate, 5 times Denhardt's solution, sonicated salmon sperm DNA (50 µg/ml), 0.1% SDS, and 10% dextran sulfate at 42 degree C., with washes at 42 degree C. in 0.2 times SSC (sodium chloride/sodium citrate) and 50% formamide at 55 degree C., followed by a high-stringency wash consisting of 0.1 times SSC containing EDTA at 55 degree C. The skilled artisan will recognize how to adjust the temperature, ionic strength, etc. as necessary to accommodate factors such as probe length and the like.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

The polynucleotides of this invention can be obtained using chemical synthesis, recombinant methods, or PCR. Methods of chemical polynucleotide synthesis are well known in the art and need not be described in detail herein. One of skill in the art can use the sequences provided herein and a commercial DNA synthesizer to produce a desired DNA sequence.

For preparing polynucleotides using recombinant methods, a polynucleotide comprising a desired sequence can be inserted into a suitable vector, and the vector in turn can be introduced into a suitable host cell for replication and amplification, as further discussed herein. Polynucleotides may be inserted into host cells by any means known in the art. Cells are transformed by introducing an exogenous polynucleotide by direct uptake, endocytosis, transfection, F-mating or electroporation. Once introduced, the exogenous polynucleotide can be maintained within the cell as a non-integrated vector (such as a plasmid) or integrated into the host cell genome. The polynucleotide so amplified can be isolated from the host cell by methods well known within the art. See, e.g., Sambrook et al. (1989).

In a preferred embodiment of the invention, the nucleotide sequence specified under d) has at least 40% homology to any of the nucleotide sequences specified under a) through c).

In another preferred embodiment of the invention, the nucleotide sequence specified under d) has at least 60%, preferably 70%, more preferably 80%, especially preferably 90% homology to any of the nucleotide sequences specified under a) through c).

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping group moieties of from 1 to 20 carbon atoms. Other hydroxyls may also be derivatized to standard protecting groups.

Polynucleotides can also contain analogous forms of ribose or deoxyribose sugars that are generally known in the art, including, for example, 2'--O-methyl-, 2'-O-allyl, 2'-fluoro- or 2'-azido-ribose, carbocyclic sugar analogs, .alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR.sub.2 ("amidate"), P(O)R', P(O)OR', CO or CH.sub.2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

In another preferred embodiment of the invention, the nucleic acid molecule of the invention is a genomic DNA, a cDNA and/or an RNA.

The invention also relates to a vector comprising the nucleic acid molecule of the invention. As used herein, "vector" means a construct, which is capable of delivering, and preferably expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

In another aspect, the invention relates to a host cell comprising the vector of the invention. A "host cell" includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. A host cell includes cells transfected in vivo with a polynucleotide(s) of this invention. The invention also relates to an animal comprising said host cell.

In yet another aspect, the invention relates to a recognition molecule or binding protein (e. g. an antibody) comprising the peptide of the invention.

In a preferred embodiment, the antibody is a camel antibody or a part thereof, for example a camelid VHH-antibody domain. The antibody of the invention advantageously binds amyloid with high affinity but shows little interactions with disaggregated, monomeric precursor polypeptides. In a preferred embodiment, the antibody possesses the remarkable property for it recognizes amyloid irrespective of the underlying polypeptide sequence and whether amyloid samples come from in vitro or in vivo sources. This is possible because the basic polypeptide conformation of amyloid is always the same. Although this conformation is a beta-sheet structure, it is distinct from beta-sheets in native proteins. The antibody of the invention does advantageously not recognize the beta-sheets in native proteins. The antibody of the invention advantageously discriminates between amyloid and native protein conformations. The antibody of the invention is so far the only protein known to do so. Diagnosis of extracerebral amyloidosis differs from diagnosis of cerebral amyloidosis, because the former are clinically far more heterogeneous and diverse in origin. By definition, amyloid can only be diagnosed by histological investigation of a tissue sample using an amyloid-specific stain to reveal the presence of amyloid. After *diagnosis* has been obtained *amyloid classification* reveals the type of amyloid (i.e. which protein forms the deposit). Currently, amyloid diagnosis requires Congo red polarising microscopy. Several severe problems are associated with this method: its low sensitivity makes early diagnosis very difficult and creates significant sampling errors with small amyloid deposits, results are non-quantitative, different laboratories use significantly different (and often incorrect) Congo red staining protocols, correct amyloid diagnosis requires a polarisation microscope that is not generally available in pathology departments. This has lead to numerous cases of misdiagnosis. Different types of amyloidosis differ by the protein forming the fibrils. Altogether there are more than 25 amyloidosis-associated proteins. Amyloid classification is performed normally with sequence-specific antibodies, but only specialised labs hold antibodies for more than a handful of amyloidoses. Moreover, some amyloidoses are notoriously difficult to classify, such as AL.

Historically, the most investigated binding proteins are antibodies. Antibodies normally display binding sites composed of two separate polypeptide chains, assembled as tetrameric protein in the immunoglobulin IgG molecule. More recently, it has become possible to produce single polypeptide chain binding proteins, in which binding is mediated by a single protein domain. Such binding proteins can be based on the same or highly related protein scaffolds or sequence, yet display highly divergent binding specificities. Preferably, the peptides of the invention are defined as a single polypeptide chain binding proteins. Frequently, they are made by first providing a certain level of diversity in a chosen monomeric protein scaffold or fold, which itself can have a natural origin or synthetic basis, and then using molecular selection or screening methods to identify amongst the protein variants those that show a desirable binding specificity. Alternatively, they are harvested from nature, such as the "heavy chain only" camelid, shark and/or llama antibodies.

The term specific recognition, specifically recognizing, specifically binding with, specifically reacting with or specifically forming an immunological reaction with refers to a binding reaction by the antibody to the amyloid fibrillar and/or non-fibrillar polypeptides or fragments thereof respectively in the sample tested, in the presence of a heterogeneous population of other proteins and/or other biologics. Thus, under the designated immunoassay conditions, the specified antibody preferentially binds to the amyloid fibrillar and/or non-fibrillar polypeptides or fragments thereof.

As used herein, an "antibody" generally refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids, primarily responsible for antigen recognition. The terms "variable light chain" and "variable heavy chain" refer to these variable regions of the light and heavy chains respectively. Optionally, the antibody or the immunological portion of the antibody, can be chemically conjugated to, or expressed as, a fusion protein with other proteins.

Antibodies of the invention include, but are not limited to polyclonal, monoclonal, bispecific, human, humanized or chimeric antibodies, single variable fragments (ssFv), single chain fragments (scFv), Fab fragments, F(ab') fragments, fragments produced by a Fab expression library, anti-idiotypic antibodies and epitope-binding fragments of any of the above, provided that they retain the original binding properties. Also mini-antibodies and multivalent antibodies such as diabodies, triabodies, tetravalent antibodies and peptabodies can be used in a method of the invention. The immunoglobulin molecules of the invention can be of any class (i.e. IgG, IgE, IgM, IgD and IgA) or subclass of immunoglobulin molecules.

Various adjuvants may be used to enhance the immunological response of the antibody of the invention, depending on the host species, including, but not limited to, complete or incomplete Freund's adjuvant, a mineral gel such as aluminum hydroxide, surface active substances such as lysolecithin, pluronic polyol, a polyanion, a peptide, an oil emulsion, keyhole limpet hemocyanin, dinitrophenol, or an adjuvant such as BCG (bacille Calmette-Guerin) or corynebacterium parvum. For the preparation of monoclonal antibodies, any technique which provides for the production of antibody molecules by continuous cell lines in culture may be used. Hyperimmunization of an appropriate donor, generally a mouse, with the antigen is undertaken. Isolation of splenic antibody producing cells is then carried out. These cells are fused to a cell characterized by immortality, such as a myeloma cell, to provide a fused cell hybrid (Hybridoma) which can be maintained in culture and which secretes the required monoclonal antibody. The cells are then cultured in bulk and the monoclonal antibodies harvested from the culture media for use. Specific techniques include but are not limited to the hybridoma technique developed by Kohler and Milstein (1975), the human B-cell hybridoma technique (Kozbor et al., 1983) or the EBV-hybridoma technique to produce human monoclonal antibodies (Cole et al., 1985). Screening for the desired antibody can be done by techniques known in the art, such as ELISA. Thus, in a particular embodiment, the present invention provides an antibody that binds with greater affinity (particularly at least 2-fold, more particularly at least 5-fold, still more particularly at least 10-fold greater affinity) to amyloid fibrillar and/or non-fibrillar polypeptides or fragments thereof than to another protein. In another preferred embodiment, the present invention provides an antibody that binds with greater affinity (particularly at least 2-fold, more particularly at least 5-fold, still more particularly at least 10-fold greater affinity) to an N-terminal truncated and/or post-translationally modified amyloid fibrillar and/or non fibrillar polypeptide or fragments thereof.

While various antibody fragments are defined in terms of enzymatic digestion of an intact antibody with papain, pepsin or other proteases, one of skill will appreciate that such antibody fragments as well as full size antibodies may be synthesized de novo either chemically or by utilizing recombinant DNA methodology. Thus, the term antibody, as used herein, also includes antibodies and antibody fragments either produced by the modification of whole antibodies or synthesized de novo using recombinant DNA methodologies.

Heteroconjugate antibodies, comprising two covalently joined antibodies, are also within the scope of the invention. Heteroconjugate antibodies may be made using any convenient cross-linking methods. Suitable cross-linking agents and techniques are well known in the art, and are described in U.S. Pat. No. 4,676,980.

Chimeric or hybrid antibodies also may be prepared in vitro using known methods of synthetic protein chemistry, including those involving cross-linking agents. For example, immunotoxins may be constructed using a disulfide exchange reaction or by forming a thioether bond. Examples of suitable reagents for this purpose include iminothiolate and methyl-4-mercaptobutyrimidate.

Humanized antibody comprising one or more CDRs of antibodies of the invention or one or more CDRs derived from said antibodies can be made using any methods known in the art. For example, four general steps may be used to humanize a monoclonal antibody. These are: (1) determining the nucleotide and predicted amino acid sequence of the starting antibody light and heavy variable domains (2) designing the humanized antibody, i.e., deciding which antibody framework region to use during the humanizing process (3) the actual humanizing methodologies/techniques and (4) the transfection and expression of the humanized antibody. See, for example, U.S. Pat. Nos. 4,816,567; 5,807,715; 5,866,692; 6,331,415; 5,530,101; 5,693,761; 5,693,762; 5,585,089; 6,180,370; 5,225,539; 6,548,640.

The term humanized antibody means that at least a portion of the framework regions of an immunoglobulin is derived from human immunoglobulin sequences. The humanized versions of the mouse monoclonal antibodies can, for example, be made by means of recombinant DNA technology, departing from the mouse and/or human genomic DNA sequences coding for H and L chains or from cDNA clones coding for H and L chains. Humanized forms of mouse antibodies can be generated by linking the CDR regions of non-human antibodies to human constant regions by recombinant DNA techniques (Queen et al., 1989; WO 90/07861). Alternatively the monoclonal antibodies used in the method of the invention may be human monoclonal antibodies. Human antibodies can be obtained, for example, using phage-display methods (WO 91/17271; WO 92/01047). In these methods, libraries of phage are produced in which members display different antibodies on their outer surfaces. Antibodies are usually displayed as Fv or Fab fragments. Phage displaying antibodies with a desired specificity are selected by affinity enrichment to amyloid fibrillar polypeptides. Human antibodies against amyloid fibrillar and/or non-fibrillar polypeptides or fragments thereof can also be produced from non-human transgenic mammals having transgenes encoding at least a segment of the human immunoglobulin locus and an inactivated endogenous immunoglobulin locus (W093/12227; WO 91/10741).

As used herein, humanized antibodies refer also to forms of non-human (e.g. murine, camel, Ilama, shark) antibodies that are specific chimeric immunoglobulins, immunoglobulin chains, or fragments thereof (such as Fv, Fab, Fab', F(ab')₂ or other antigen-binding subsequences of antibodies) that contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework region (FR) residues of the human immunoglobulin are replaced by corresponding non-human residues. Furthermore, the humanized antibody may comprise residues that are found neither in the recipient antibody nor in the imported CDR or framework sequences, but are included to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin. Antibodies may have Fc regions modified as described in WO 99/58572. Other forms of humanized antibodies have one or more CDRs (one, two, three, four, five, six) which are altered with respect to the original antibody, which are also termed one or more CDRs "derived from" one or more CDRs (e. g. SEQ ID No. 2, 3 and/or 4) from the original antibody, preferably a camel antibody with the sequence of SEQ ID No. 1 and CDRs according to SEQ ID No. 2, 3 and/or 4.

As used herein, human antibody means an antibody having an amino acid sequence corresponding to that of an antibody produced by a human and/or has been made using any of the techniques for making human antibodies known in the art or disclosed herein. This definition of a human antibody includes antibodies comprising at least one human heavy chain polypeptide or at least one human light chain polypeptide. One such example is an antibody comprising murine light chain and human heavy chain polypeptides. Human antibodies can be produced using various techniques known in the art. In one embodiment, the human antibody is selected from a phage library, where that phage library expresses human antibodies (Vaughan et al., 1996, Nature Biotechnology, 14:309-314; Sheets et al., 1998, PNAS, (USA) 95:6157-6162; Hoogenboom and Winter, 1991, J. Mol. Biol., 227:381; Marks et al., 1991, J. Mol. Biol., 222:581). Human antibodies can also be made by introducing human immunoglobulin loci into transgenic animals, e.g., mice in which the endogenous immunoglobulin genes have been partially or completely inactivated. This approach is described in U.S. Pat. Nos. 5,545,807; 5,545,806; 5,569,825; 5,625,126; 5,633,425; and 5,661,016. Alternatively, the human antibody may be prepared by immortalizing human B lymphocytes that produce an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boemer et al., 1991, J. Immunol., 147 (1):86-95; and U.S. Pat. No. 5,750,373.

Human antibodies can be selected by competitive binding experiments, or otherwise, to have the same epitope specificity as a particular mouse antibody. Such antibodies are particularly likely to share the useful functional properties of the mouse antibodies. Human polyclonal antibodies can also be provided in the form of serum from humans immunized with an immunogenic agent. Optionally, such polyclonal antibodies can be concentrated by affinity purification using amyloid fibrillar and/or non-fibrillar polypeptides or fragments thereof as an affinity reagent. Monoclonal antibodies can be obtained from serum according to the technique described in WO 99/60846.

A variable region of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. There are at least two techniques for determining CDRs: (1) an approach based on cross-species sequence variability (i.e., Kabat et al. Sequences of Proteins of Immunological Interest, (5th ed., 1991, National Institutes of Health, Bethesda Md.)); and (2) an approach based on crystallographic studies of antigen-antibody complexes (Allazikani et al (1997) J. Molec. Biol. 273:927-948)). As used herein, a CDR may refer to CDRs defined by either approach or by a combination of both approaches.

A "constant region" of an antibody refers to the constant region of the antibody light chain or the constant region of the antibody heavy chain, either alone or in combination.

The term "Fc region" is used to define a C-terminal region of an immunoglobulin heavy chain. The "Fc region" may be a native sequence Fc region or a variant Fc region. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The numbering of the residues in the Fc region is that of the EU index as in Kabat. Kabat et al., Sequences of Proteins of Imunological Interest, 5th Ed. Public Health Service, National Institutes of Health, Bethesda, Md., 1991. The Fc region of an immunoglobulin generally comprises two constant domains, CH2 and CH3.

As used herein, "Fc receptor" and "FcR" describe a receptor that binds to the Fc region of an antibody. The preferred FcR is a native sequence human FcR. Moreover, a preferred FcR is one which binds an IgG antibody (a gamma receptor) and includes receptors of the Fc.gamma.RI, Fc.gamma.RII, and Fc.gamma.RIII subclasses, including allelic variants and alternatively spliced forms of these receptors. Fc.gamma.RII receptors include Fc.gamma.RIIA (an "activating receptor") and Fc.gamma.RIIB (an "inhibiting receptor"), which have similar amino acid sequences that differ primarily in the cytoplasmic domains thereof. FcRs are reviewed in Ravetch and Kinet, 1991, Ann. Rev. Immunol., 9:457-92; Capel et al., 1994, Immunomethods, 4:25-34; and de Haas et al., 1995, J. Lab. Clin. Med., 126:330-41. "FcR" also includes the neonatal receptor, FcRn, which is responsible for the transfer of maternal IgGs to the fetus (Guyer et al., 1976, J. Immunol., 117:587; and Kim et al., 1994, J. Immunol., 24:249). Complement dependent cytotoxicity" and "CDC" refer to the lysing of a target in the presence of complement. The complement activation pathway is initiated by the binding of the first component of the complement system (Clq) to a molecule (e.g. an antibody) complexed with a cognate antigen. To assess complement activation, a CDC assay, e.g. as described in Gazzano-Santoro et al., J. Immunol. Methods, 202:163 (1996), may be performed. A "functional Fc region" possesses at least one effector function of a native sequence Fc region. Exemplary "effector functions" include Clq binding; complement dependent cytotoxicity (CDC); Fc receptor binding; antibody-dependent cell-mediated cytotoxicity (ADCC); phagocytosis; down-regulation of cell surface receptors (e.g. B cell receptor; BCR), etc. Such effector functions generally require the Fc region to be combined with a binding domain (e.g. an antibody variable domain) and can be assessed using various assays known in the art for evaluating such antibody effector functions. A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification, yet retains at least one effector function of the native sequence Fc region. Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95%, at least about 96%, at least about 97%, at least about 98%, at least about 99% sequence identity therewith.

As indicated above, the present invention also provides preparations and methods for preventing and treating, in a mammal, a disease characterized by amyloid formation and/or aggregation, preferably by promoting a non-fibrillar aggregation or in another preferred embodiment by induction of an immune response in a mammal or human patient. Accordingly, the present invention also provides a vaccine composition or a therapeutic composition comprising a peptide of the invention as referred to above, comprising an additional antibody or a T-cell specific for an N-terminal truncated and/or post-translationally modified A beta peptide, or comprising a nucleic acid encoding an N-terminal truncated post-translationally modified A beta peptide and/or amyloid fibrillar/non-fibrillar polypeptides.

As used herein, the term "preventing a disease" means inhibiting or reversing the onset of the disease, inhibiting or reversing the initial signs of the disease (i.e. formation and/or aggregation of amyloid peptides), inhibiting the appearance of clinical symptoms of the disease. As used herein, the term "treating a disease" includes substantially inhibiting the disease, substantially slowing or reversing the progression of the disease, substantially ameliorating clinical symptoms of the disease or substantially preventing the appearance of clinical symptoms of the disease.

The mammal examined in the present invention may be a non-human mammal, such as (but not limited to) a cow, a pig, a sheep, a goat, a horse, a monkey, a rabbit, a hare, a dog, a cat, a mouse, a rat, an elk, a deer, or a tiger. In a preferred embodiment, the mammal is a primate. In another preferred embodiment the mammal is a human, more preferably the mammal is a human adult.

The vaccine or therapeutic compositions of the present invention induce an immune response against the amyloid fibrillar and/or non-fibrillar polypeptides or fragments thereof. The induction of an immune response is "active" when an immunogen is administered to induce antibodies or T-cells reactive against the immunogen. The induction of an immune response is "passive" when an antibody is administered that itself binds to the amyloid fibrillar and/or non-fibrillar polypeptides or fragments thereof in the mammal.

In a preferred embodiment of the invention, the recognition-molecule is an antibody-fragment of an antibody or an antibody.

In another preferred embodiment of the invention, said antibody or the fragment thereof is a chimeric antibody or a fragment thereof.

In another preferred embodiment of the invention, said chimeric antibody or the fragment thereof is a (partially) humanized antibody or a fragment thereof.

In another preferred embodiment of the invention, said fragment of the antibody is a variable fragment of a single-chain of an antibody.

In another preferred embodiment of the invention, said single-chain is a heavy chain variable domain.

Preferably, the antibody of the invention comprises the sequences SEQ ID No. 2, 3 or 4, e. g. as CDR-region.

The CDR portions of the antibody according to SEQ ID No. 1 are preferably SEQ ID No. 2, 3 and/or 4 or other parts of the sequence. Determination of CDR regions is well within the skill of the art. It is understood that in some embodiments, CDRs can be a combination of the Kabat and Chothia CDR (also termed "combined CDRs" or "extended CDRs"). In some embodiments, the CDRs are the Kabat CDRs. In other embodiments, the CDRs are the Chothia CDRs. In other words, in embodiments with more than one CDR, the CDRs may be any of Kabat, Chothia, combination CDRs, or combinations thereof.

In some embodiments, the invention provides a polypeptide (which may or may not be an antibody) which comprises at least one CDR, at least two, at least three, or more CDRs that are substantially identical to at least one CDR, at least two, at least three, or more CDRs of the antibody or peptide of the invention. Other embodiments include antibodies which have at least two, three, four, five, or six CDR(s) that are substantially identical to at least two, three, four, five or six CDRs of the antibodies of the invention or derived from the antibodies of the invention. In some embodiments, the at least one, two, three, four, five, or six CDR(s) are at least about 85%, 86%, 87%, 88%, 89%, 90%, 95%, 96%, 97%, 98%, or 99% identical to at least one, two or three CDRs of the antibody of the invention. It is understood that, for purposes of this invention, binding specificity and/or overall activity is generally retained, although the extent of activity may vary compared to said antibody (may be greater or lesser).

The invention also provides a polypeptide (which may or may not be an antibody) which comprises an amino acid sequence of SEQ ID No. 1, 2, 3 and/or 4 or its variants that has any of the following: at least 3 contiguous amino acids, at least 4 contiguous amino acids, at least about 5 contiguous amino acids, at least about 10 contiguous amino acids, at least about 15 contiguous amino acids, at least about 20 contiguous amino acids, at least about 25 contiguous amino acids of a sequence of SEQ ID No. 1 or its variants, wherein at least 3 of the amino acids are from a sequence of SEQ ID No. 2, 3 and/or 4 or its variants. In a preferred embodiment, the sequences of SEQ ID No. 2, 3 or 4 represent the variable region of the antibody, more preferably the CDR-region. In one embodiment, the variable region is from a light chain, in another embodiment, the variable region is from a heavy chain of the antibody.

The binding affinities of the antibodies and polypeptides of the invention may vary, and need not be (but can be) a particular value or range, as the exemplary embodiments described below. The binding affinity (K_{D}) of the antibodies and polypeptides of the invention to amyloid fibrillar and/or non-fibrillar polypeptides can be about 0.10 to about 0.80 nM, about 0.15 to about 0.75 nM and about 0.18 to about 0.72 nM. In some embodiments, the binding affinity is about 2 pM, about 5 pM, about 10 pM, about 15 pM, about 20 pM, about 40 pM, or greater than about 40 pM. In one embodiment, the binding affinity is between about 2 pM and 22 pM. In other embodiments, the binding affinity is less than about 10 nM, about 5 nM, about 4 nM, about 3 nM, about 2 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM. In some embodiment, the binding affinity is about 10 nM. In other embodiments, the binding affinity is less than about 10 nM, less than about 50 nM, less than about 100 nM, less than about 150 nM, less than about 200 nM, less than about 250 pM, less than about 500 nM, or less than about 1000 nM. In other embodiments, the binding affinity is less than about 5 nM. In other embodiments, the binding affinity is less than about 1 nM. In other embodiments, the binding affinity is about 0.1 nM or about 0.07 nM. In other embodiments, the binding affinity is less than about 0.1 nM or less than about 0.07 nM. In other embodiments, the binding affinity is from any of about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM to any of about 2 pM, about 5 pM, about 10 pM, about 15 pM, about 20 pM, or about 40 pM. In some embodiments, the binding affinity is any of about 10 nM, about 5 nM, about 1 nM, about 900 pM, about 800 pM, bout 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM. In still other embodiments, the binding affinity is about 2 pM, about 5 pM, about 10 pM, about 15 pM, about 20 pM, about 40 pM, or greater than about 40 pM. In a more preferred embodiment, the binding affinity is about 7 nM.

The invention encompasses modifications to the antibody of the invention, including functionally equivalent antibodies which do not significantly affect their properties and variants which have enhanced or decreased activity and/or affinity. For example, amino acid sequence of the antibody may be mutated to obtain an antibody with the desired binding affinity to amyloid polypeptides. Modification of polypeptides of the invention is routine practice in the art and need not be described in detail herein. Examples of modified polypeptides include polypeptides with conservative substitutions of amino acid residues, one or more deletions or additions of amino acids which do not significantly deleteriously change the functional activity, or use of chemical analogs.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to an epitope tag. Other insertional variants of the antibody molecule include the fusion to the N- or C-terminus of the antibody of an enzyme or a polypeptide which increases the serum half-life of the antibody.

Substitution variants have at least one amino acid residue in the antibody molecule removed and a different residue inserted in its place. The sites of greatest interest for substitutional mutagenesis include the hypervariable regions, but FR alterations are also contemplated. If such substitutions result in a change in biological activity, then more substantial changes, denominated "exemplary substitutions" in Table 1, or as further described below in reference to amino acid classes, may be introduced and the products screened.

**TABLE 1 Amino Acid Substitutions**

| **Original Residue** | **Conservative Substitutions** | **Exemplary Substitutions** |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) Non-polar: Norleucine, Met, Ala, Val, Leu, Ile;
(2) Polar without charge: Cys, Ser, Thr, Asn, Gln;
(3) Acidic (negatively charged): Asp, Glu;
(4) Basic (positively charged): Lys, Arg;
(5) Residues that influence chain orientation: Gly, Pro; and
(6) Aromatic: Trp, Tyr, Phe, His.

Non-conservative substitutions are made by exchanging a member of one of these classes for another class.

Any cysteine residue not involved in maintaining the proper conformation of the antibody also may be substituted, generally with serine, to improve the oxidative stability of the molecule and prevent aberrant cross-linking. Conversely, cysteine bond(s) may be added to the antibody to improve its stability, particularly where the antibody is an antibody fragment such as an Fv fragment.

Amino acid modifications can range from changing or modifying one or more amino acids to complete redesign of a region, such as the variable region. Changes in the variable region can alter binding affinity and/or specificity. In some embodiments, no more than one to five conservative amino acid substitutions are made within a CDR domain. In other embodiments, no more than one to three conservative amino acid substitutions are made within a CDR domain.

Modifications also include glycosylated and nonglycosylated polypeptides, as well as polypeptides with other post-translational modifications, such as, for example, glycosylation with different sugars, acetylation, and phosphorylation. Antibodies are glycosylated at conserved positions in their constant regions (Jefferis and Lund, 1997, Chem. Immunol. 65:111-128; Wright and Morrison, 1997, TibTECH 15:26-32). The oligosaccharide side chains of the immunoglobulins affect the protein's function (Boyd et al., 1996, Mol. Immunol. 32:1311-1318; Wittwe and Howard, 1990, Biochem. 29:4175-4180) and the intramolecular interaction between portions of the glycoprotein, which can affect the conformation and presented three-dimensional surface of the glycoprotein (Hefferis and Lund, supra; Wyss and Wagner, 1996, Current Opin. Biotech. 7:409-416). Oligosaccharides may also serve to target a given glycoprotein to certain molecules based upon specific recognition structures. Glycosylation of antibodies has also been reported to affect antibody-dependent cellular cytotoxicity (ADCC). In particular, CHO cells with tetracycline-regulated expression of beta(1,4)-N-acetylglucosaminyltransferase III (GnTIII), a glycosyltransferase catalyzing formation of bisecting GlcNAc, was reported to have improved ADCC activity (Umana et al., 1999, Mature Biotech. 17:176-180).

Glycosylation of antibodies is typically either N-linked or O-linked. N-linked refers to the attachment of the carbohydrate moiety to the side chain of an asparagine residue. The tripeptide sequences asparagine-X-serine, asparagine-X-threonine, and asparagine-X-cysteine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be used.

The following methods may be used for adjusting the affinity of an antibody and for characterizing a CDR. One way of characterizing a CDR of an antibody and/or altering (such as improving) the binding affinity of a polypeptide, such as an antibody, termed "library scanning mutagenesis". Generally, library scanning mutagenesis works as follows. One or more amino acid positions in the CDR are replaced with two or more (such as 3, 4, 5, 6, 7, 8, 9, 10, 11, 12, 13, 14, 15, 16, 17, 18, 19, or 20) amino acids using art recognized methods. This generates small libraries of clones (in some embodiments, one for every amino acid position that is analyzed), each with a complexity of two or more members (if two or more amino acids are substituted at every position). Generally, the library also includes a clone comprising the native (unsubstituted) amino acid. A small number of clones, e.g., about 20-80 clones (depending on the complexity of the library), from each library are screened for binding affinity to the target polypeptide (or other binding target), and candidates with increased, the same, decreased or no binding are identified. Methods for determining binding affinity are well-known in the art. Binding affinity may be determined using BIAcore surface plasmon resonance analysis, which detects differences in binding affinity of about 2-fold or greater. BIAcore is particularly useful when the starting antibody already binds with a relatively high affinity, for example a K.sub.D of about 10 nM or lower. Screening using BIAcore surface plasmon resonance is described in the Examples, herein.

Binding affinity may be determined using Kinexa Biocensor, scintillation proximity assays, ELISA, ORIGEN immunoassay (IGEN), fluorescence quenching, fluorescence transfer, and/or yeast display. Binding affinity may also be screened using a suitable bioassay.

In some embodiments, every amino acid position in a CDR is replaced (in some embodiments, one at a time) with all 20 natural amino acids using art recognized mutagenesis methods (some of which are described herein). This generates small libraries of clones (in some embodiments, one for every amino acid position that is analyzed), each with a complexity of 20 members (if all 20 amino acids are substituted at every position).

In some embodiments, the library to be screened comprises substitutions in two or more positions, which may be in the same CDR or in two or more CDRs. Thus, the library may comprise substitutions in two or more positions in one CDR. The library may comprise substitution in two or more positions in two or more CDRs. The library may comprise substitution in 3, 4, 5, or more positions, said positions found in two, three, four, five or six CDRs. The substitution may be prepared using low redundancy codons. See, e.g., Table 2 of Balint et al., (1993) Gene 137(1):109-18).

The CDR may be CDRH3 and/or CDRL3. The CDR may be one or more of CDRL1, CDRL2, CDRL3, CDRH1, CDRH2, and/or CDRH3. The CDR may be a Kabat CDR, a Chothia CDR, or an extended CDR.

Candidates with improved binding may be sequenced, thereby identifying a CDR substitution mutant which results in improved affinity (also termed an "improved" substitution). Candidates that bind may also be sequenced, thereby identifying a CDR substitution which retains binding.

Multiple rounds of screening may be conducted. For example, candidates (each comprising an amino acid substitution at one or more position of one or more CDR) with improved binding are also useful for the design of a second library containing at least the original and substituted amino acid at each improved CDR position (i.e., amino acid position in the CDR at which a substitution mutant showed improved binding). Preparation, and screening or selection of this library is well known in the state of the art.

Library scanning mutagenesis also provides a means for characterizing a CDR, in so far as the frequency of clones with improved binding, the same binding, decreased binding or no binding also provide information relating to the importance of each amino acid position for the stability of the antibody-antigen complex. For example, if a position of the CDR retains binding when changed to all 20 amino acids, that position is identified as a position that is unlikely to be required for antigen binding. Conversely, if a position of CDR retains binding in only a small percentage of substitutions, that position is identified as a position that is important to CDR function. Thus, the library scanning mutagenesis methods generate information regarding positions in the CDRs that can be changed to many different amino acid (including all 20 amino acids), and positions in the CDRs which cannot be changed or which can only be changed to a few amino acids.

Candidates with improved affinity may be combined in a second library, which includes the improved amino acid, the original amino acid at that position, and may further include additional substitutions at that position, depending on the complexity of the library that is desired, or permitted using the desired screening or selection method. In addition, if desired, adjacent amino acid position can be randomized to at least two or more amino acids. Randomization of adjacent amino acids may permit additional conformational flexibility in the mutant CDR, which may in turn, permit or facilitate the introduction of a larger number of improving mutations. The library may also comprise substitution at positions that did not show improved affinity in the first round of screening.

The second library is screened or selected for library members with improved and/or altered binding affinity using any method known in the art, including screening using BIAcore surface plasmon resonance analysis, and selection using any method known in the art for selection, including phage display, yeast display, and ribosome display.

The invention also relates to solid phases for affinity chromatography or solid-phase extraction consisting of organic, inorganic, synthetic polymers or of mixed polymers, preferably cross-linked agarose, cellulose, silica gel, polyamide and polyvinyl alcohols, which are optionally chemically activated, with peptides or a recognition-molecule of the invention immobilized on the surface of the solid phase.

In a preferred embodiment of the invention, the peptides of the solid phases are bound to the solid support phase covalently or by adsorption.

In another preferred embodiment of the invention, the peptides of the solid phases are covalently bound to the solid phase on any of positions X1 to X135.

In another preferred embodiment of the invention, the peptides of the solid phases are distanced from the support surface by linkers/spacers.

The invention also relates to a device for removing amyloid fibrillar and/or non-fibrillar polypeptides from samples on solid phases, wherein the device contains a solid phase of the invention, and means for the entry of samples are provided.

In yet another aspect, the invention relates to a pharmaceutical composition comprising a nucleic acid molecule according to the invention, a vector of the invention, a host cell of the invention, a peptide according to the invention, a recognition-molecule according to the invention and/or a solid phase according to the invention; optionally together with a pharmaceutically tolerable carrier.

In a preferred embodiment of the invention, the pharmaceutical agent of the invention comprises a carrier selected from the group comprising fillers, disintegrants, binders, humectants, extenders, dissolution retarders, absorption enhancers, wetting agents, adsorbents and/or lubricants.

In another preferred embodiment of the invention, said agent is a capsule, a tablet, a coated tablet, a suppository, an ointment, a cream, an injection solution and/or an infusion solution.

In another preferred embodiment of the invention, said agent is an oral, vaginal, rectal, nasal, topical, subcutaneous, intravenous, intramuscular, intraperitoneal agent, suppository, pad and/or foam.

The invention also relates to a kit comprising a nucleic acid molecule according to the invention, a vector according to the invention, a host cell according to the invention, a peptide according the invention, a recognition-molecule according to the invention, a solid phase according to the invention and/or a pharmaceutical composition according to the invention, optionally together with instructions for combining the contents of the kit and/or providing a formulation. The kits may comprise, in suitable container means, one or more binding peptides and/or antibodies, a control protein or peptide (e.g., A beta 42 or amyloid polypeptides), a control peptide that is not recognized by the antibody of the invention and various buffers, reagents, enzymes and other standard ingredients well known in the art.

The container means of the kits will generally include at least one vial, test tube, flask, bottle, syringe or other container means, into which the probes and/or primers may be placed, and preferably, suitably aliquoted. Where an additional component is provided, the kit will also generally contain additional containers into which this component may be placed. The kits of the present invention will also typically include a means for containing the peptides or proteins and any other reagent containers in close confinement for commercial sale. Such containers may include injection or blow-molded plastic containers into which the desired vials are retained.

In a preferred embodiment of the invention, the kit is used for the therapy of amyloid diseases.

In another preferred embodiment of the invention, the kit is used for in vitro or in vivo diagnosis of amyloid diseases.

In another aspect, the kit of the invention is used as an amyloid-histology kit, an amyloid-ELISA kit, an amyloid affinity kit and/or as an amyloid classification kit. The binder, that means the peptide or the recognition-molecule, e. g. antibody of the invention, interacts with a structural epitope, common and specific to all amyloid fibrils. The peptides and especially the antibody of the invention can advantageously be used as conformation-sensitive amyloid binders. The conformation-dependent binders of the invention (e. g. the antibody) can advantageously be used in the non-invasive in vivo amyloid diagnosis or for amyloid therapy. Since extracerebral amyloidoses primarily affect parenchymal organs such as heart, liver and kidney, these deposits are also easier to reach diagnostically and therapeutically than those from cerebral amyloidoses (no blood-brain barrier, extracellular deposition). The antibody of the invention has a significantly higher amyloid-affinity than molecules of the state of the art. The kit of the invention allows a prescreen of patients before taking biopsies and for readily evaluating therapeutic strategies. These assays of the invention are based on a protein that binds a conformational epitope specific for and common to all amyloid fibrils. Its phosphatase moiety allows direct detection in histology and ELISA and signal amplifications, wherein a secondary antibody is not needed.

In prophylactic applications, pharmaceutical or vaccine compositions are administered to a mammal susceptible to, or at risk of developing a disease associated with amyloid formation and/or aggregation in an amount sufficient to eliminate or reduce the risk or delay the ontset of said disease. In therapeutic applications, compositions are administered to a mammal suspected of, or already suffering from such a disease in an amount sufficient to cure, or at least partially arrest, the symptoms of the disease and its complications. An amount adequate to accomplish this is defined as a therapeutically- or pharmaceutically-effective dose. In both prophylactic and therapeutic regimes, agents are usually administered in several dosages until a sufficient immune response has been achieved. Typically, the immune response is monitored and repeated dosages are given if the immune response starts to fade.

For passive immunization with an antibody, the dosage ranges from about 0.0001 to 100 mg/kg, and more usually 0.01 to 5 mg/kg of the host body weight. Doses for nucleic acids encoding the peptide/recognition-molecule of the invention range from about 10 ng to 1 g, 100 ng to 100 mg, 1 µg to 10 mg, or 30 to 300 µg DNA per patient. Doses for infectious viral vectors vary from 10 to 10.sup.9 or more virions per dose. Effective doses of the therapeutic compositions (e. g. vaccine) of the present invention, vary depending upon many different factors, including means of administration, target site, physiological state of the mammal, whether the patient is a human or an animal, other medications administered, and whether treatment is prophylactic or therapeutic. Treatment dosages need to be titrated to optimize safety and efficacy. The amount of peptides/recognition molecules of the invention depends on whether adjuvant is also administered, with higher dosages being required in the absence of adjuvant. The amount of peptides/recognition molecules of the invention for administration sometimes vary from 1-500 µg per mammal and more usually from 5-500 µg per injection for human administration. Occasionally, a higher dose of 1-2 mg per injection is used. Typically about 10, 20, 50 or 100 µg is used for each human injection. The timing of injections can vary significantly from once a day, to once a year, to once a decade. On any given day that a dosage of the peptides/recognition molecules of the invention is given, the dosage is greater than 1 µg/patient and usually greater than 10 µg/patient if adjuvant is also administered. In the absence of adjuvant, the dosage is greater than 10 µg/patient and usually greater than 100 µg/patient.

Agents for inducing an immune response (e. g. antibody for passive immunization) can be administered by parenteral, topical, intravenous, oral, subcutaneous, intraperitoneal, intranasal, or intramuscular means for prophylactic and/or therapeutic treatment. The most typical route of administration is subcutaneous, although others can be equally effective. The next most common is intramuscular injection. This type of injection is most typically performed in the arm or leg muscles. Intravenous injections as well as intraperitoneal injections, intraarterial, intracranial, or intradermal injections are also effective in generating an immune response. In some methods, agents are injected directly into a particular tissue where deposits are accumulated.

Therapeutic compositions of the invention can optionally comprise other agents that are at least partly effective in treatment of diseases associated with amyloid formation and/or aggregation. In the case of Alzheimer's and Down's syndrome, in which amyloid aggregation occurs in the brain, the vaccine or therapeutic composition of the invention may also comprise other agents that increase passage of the active components of the composition of the invention across the blood-brain barrier. A peptide, protegrin PG-1, belonging to the family of beta-stranded antimicrobil peptides, for example, has been successfully used to deliver therapeutic compounds into eucaryotic cells (Drin and Temsamani, 2002). Other strategies to enhance drug delivery across the blood brain barrier, especially vector-mediated strategies, have been reviewed by Temsamani et al. (2001).

The preferred form of the vaccine and/or therapeutic composition depends on the intended mode of administration and application. The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the combination. Examples of such diluents are distilled water, physiological phosphate-buffered saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

The vaccine or pharmaceutical compositions can also include large, slowly metabolized macromolecules such as proteins, polysaccharides, polylactic acids, polyglycolic acids and copolymers (such as latex functionalized sepha-rose, agarose, cellulose, and the like), polymeric amino acids, amino acid copolymers, and lipid aggregates (such as oil droplets or liposomes). Additionally, these carriers can function as immunostimulating agents (i.e., adjuvants).

For parenteral administration, the compositions of the invention can be administered as injectable dosages of a solution or suspension of the substance in a physiologically acceptable diluent with a pharmaceutical carrier that can be a sterile liquid such as water, oils, saline, glycerol, or ethanol. Also encapsulation into biodegradable microparticles can be used as a parenteral delivery system (Brayden et al., 2001).

Additionally, auxiliary substances, such as wetting or emulsifying agents, surfactants, pH buffering substances and the like can be present in the vaccine and/or therapeutic compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions.

Typically, compositions are prepared as injectables, either as liquid solutions or suspensions. Solid forms suitable for solution in, or suspension in, liquid vehicles prior to injection can also be prepared. The preparation also can be emulsified or encapsulated in liposomes or micro particles such as polylactide, polygly-colide, or copolymer for enhanced adjuvant effect, as discussed above (Langer, 1990; Langer et al, 1997). The compositions of this invention can be administered in the form of a depot injection or implant preparation which can be formulated in such a manner as to permit a sustained or pulsatile release of the active ingredient.

Additional formulations suitable for other modes of administration include oral, intranasal, and pulmonary formulations, suppositories, and transdermal applications. For suppositories, binders and carriers include, for example, polyalkylene glycols or triglycerides. Such suppositories can be formed from mixtures containing the active ingredient in the range of 0.5% to 10%, preferably 1% to 2%. Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained release formulations, or powders and contain 10% to 95% of active ingredient, preferably 25% to 70%.

Alternatively, transdermal delivery can be achieved using a skin path or using transferosomes (Paul et al., 1995; Cevc et al., 1998).

Further techniques for formulation and administration of drugs can also be found in "Remington's Pharmaceutical Sciences".

The vaccine and therapeutic compositions of the present invention can thus be used for the prevention and/or treatment of any disease associated with amyloid formation and/or aggregation. In a preferred embodiment the vaccine and therapeutic compositions of the invention are used for the prevention and/or treatment of Alzheimer's disease, Down's syndrome or other amyloid diseases.

In non-demented humans, prophylaxis can begin at any age (e.g., 10, 20, 30 years). Usually, however, it is not necessary to begin prophylaxis until a patient reaches the age of 40, 50, 60, or 70 years. Prophylaxis typically encompasses multiple dosages over a period of time. If the response falls, a further dosage is indicated.

Accordingly, the present invention also encompasses a method for the prevention and/or treatment, in an mammal, of a disease associated with beta-amyloid formation and/or aggregation, said method comprising the administration, to said mammal, of a vaccine composition or a therapeutic composition as described above.

In another aspect, the invention also relates to an apparatus for chromatography, comprising the recognition-molecule according to the invention.

In a preferred embodiment, the recognition-molecules of the apparatus are bound to a solid phase according to the invention.
The invention also relates to the use of a nucleic acid molecule according to the invention, a vector according to the invention, a host cell according to the invention, a peptide according to the invention, a recognition-molecule according to the invention, a solid phase according to claims the invention, a pharmaceutical composition according to the invention, a kit according to the invention, an apparatus according to the invention in the prophylaxis, diagnosis, therapy, follow-up and/or aftercare of amyloid diseases. In a preferred embodiment of the invention, said amyloid diseases are selected from the group comprising
- Systemic amyloidosis
   - Primary amyloidosis
      ■ Mutations in lysozyme, transthyretin, apolipoprotein B, fibrinogen
      ■ AL amyloidosis (immunoglobulin light chains, as seen with multiple myeloma)
   - Secondary amyloidosis
      ■ AA amyloidosis (serum amyloid A protein, an acute-phase protein due to chronic inflammation)
      ■ Gelsolin amyloidosis (plasma gelsolin fragments).
   - Familial or Hereditary amyloidosis
      ■ Most commonly caused by mutations in the transthyretin protein, but in rare occurrences can also be caused by apolipoprotein Al, gelsolin, fibrinogen, and lysozyme mutations.
      ■ Primarily caused by genetics, believed to be autosomal dominant, high probability of passage to offspring
      ■ Appalachian type amyloidosis
      ■ Shar Pei fever for amyloidosis in Shar Peis
- Organ-specific amyloidosis
   - Familial or Hereditary amyloidosis
   - Diabetes mellitus type 2 (amylin, also known as IAPP)
   - Neurology
      ■ Alzheimer's disease (Aβ 39-42)
      ■ Parkinson's disease (alpha-synuclein)
      ■ Huntington's disease (huntingtin)
      ■ Transmissible spongiform encephalopathies (prion protein, PrP)
         - Creutzfeldt-Jakob disease (PrP in cerebrum)
         - Kuru (diffuse PrP deposits in brain)
         - Fatal Familial Insomnia (PrP in thalamus)
         - Bovine spongiform encephalopathy (PrP in cerebrum of cows)
      ■ Congophilic angiopathy (Amyloid beta)
   - Cardiac amyloidosis
      ■ congestive heart failure; some instances (PrP or transthyretin in heart)
   - Inclusion body myositis
- Iatrogenic conditions
   - insulin amyloidosis (injection-administered insulin)

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypo-dermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

In yet another aspect, the invention relates to a method for the treatment of the above-referenced amyloid diseases by binding and/or removal of the amyloid fibrillar polypeptides or the non-fibrillar polypeptides by means of the recognition-molecules according to the invention.

### EXAMPLES

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

N-biotinylated, disaggregated Aβ(1-40) peptide or Aβ(1-40) fibrils were captured on different flow cells of a SA sensor chip (Biacore). 22C4 and B10AP were injected in 10 mM HEPES buffer (pH 7.4), 0.15 M NaCl, 3 mM ethylenediamine-tetraacetic acid, 5 µM surfactant P20 at a flow rate of 10 µl/min. Regeneration was done with 10 mM glycine/HCl, 1 M NaCl (pH 1.5) for 1 min. K_{D}s were calculated from the concentration dependent steady-state binding of B10 or B10AP using the 1:1 steady state affinity model. Molar stoichiometry of the antibody/Aβ(1-40) fibril complexes was estimated from the measured maximum binding capacity of the fibril coated sensor surface, the response level of immobilized Aβ fibrils and the molecular mass of the antibody (15725 Da) and Aβ(1-40) (4330 Da) .

Polypeptide was blotted onto 0,45 µm nitrocellulose membrane (GE Healthcare or Schleicher & Schuell) and blocked with 2 % bovine serum albumin in Tris-buffered saline (pH 7.4, TBS) at room temperature for 1 h. Equal loading was confirmed with Ponceau red or Coomassie staining of control membranes. Membranes were washed 2 x in H₂O and 1 x in TBST (TBS with 0.1 % Tween 20) for 5 min, before they were incubated for 1 h at room temperature with 0.5 µg/ml of the antibodyAP in TBS. Competition studies were performed by preincubation of the antibodyAP for 1 h with Aβ(1-40) fibrils at 1:0.5, 1:5, 1:10 w/w ratios of Aβ to AA or AL fibrils. After the antibodyAP incubation, membranes were washed 2 x with H₂O and 1 x TBST for 5 min. Alternatively, washing and blocking may be performed in TBS containing 300 mM NaCl. The antibodyAP binding was visualised with NBT/BCIP (Pierce). Densitometric quanti-fications used TotalLab 100 software.

The figures show the following:

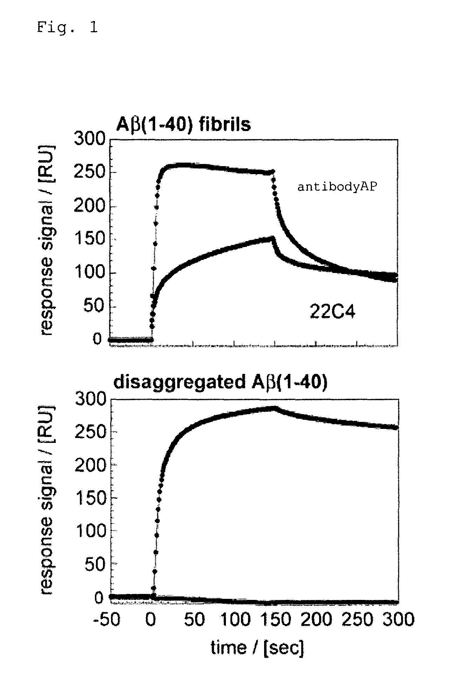

- Fig. 1:: Surface plasmon resonance sensorgrams of 100 nM 22C4 or antibodyAP to biosensors covered with fibrils or disaggregated Aβ(1-40). In the lower panel the blue curve is scaled to 1/10th of its original value.

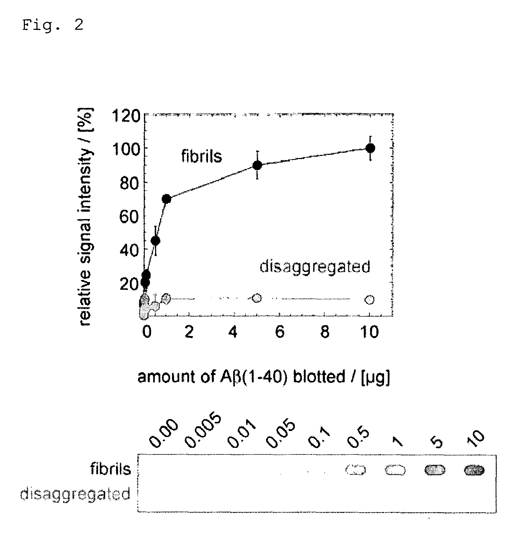

- Fig. 2:: AntibodyAP staining of disaggregated and fibrillar Aβ(1-40) blotted onto nitrocellulose, quantitated by densitometry with standard error from 3 experiments.

## Claims

1. A peptide,
**characterized in that**
it is selected from the group comprising:
a) a peptide consisting of the amino acid sequence according to SEQ ID No. 1, particularly according to SEQ ID No. 2, 3 and/or 4;
b) a peptide consisting of an amino acid sequence having sufficient homology to be functionally analogous/equivalent to an amino acid sequence in accordance with a);
c) a peptide according to an amino acid sequence a) or b) which is modified by deletions, additions, substitutions, translocations, inversions and/or insertions and functionally analogous/equivalent to an amino acid sequence in accordance with a) or b);
d) a peptide according to amino acid sequence a), b) or c) which is modified by branch or extension with the same or another peptide according to amino acid sequence a), b) or c) to form a homooligomeric or heterooligomeric peptide.

2. The peptide according to claim 1,
**characterized in that**
the amino acid sequence specified under b) has at least 40% homology to any of the amino acid sequences specified under a).

3. The peptide according to claim 1 or 2,
**characterized in that**
the amino acid sequence specified under b) has at least 60%, preferably 70%, more preferably 80%, especially preferably 90% homology to any of the amino acid sequences specified under a).

4. The peptide according to any of claims 1 to 3,
**characterized in that**
it consists of the amino acid sequence SEQ ID No. 1, 2, 3 and/or 4.

5. The peptide according to any of claims 1 to 4 comprising an amino acid sequence X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36-X37-X38-X39-X40-X41-X42-X43-X44-X45-X46-X47-X48-X49-X50-X51-X52-X53-X54-X55-X56-X57-X58-X59-X60-X61-X62-X63-X64-X65-X66-X67-X68-X69-X70-X71-X72-X73-X74-X75-X76-X77-X78-X79-X80-X81-X82-X83-X84-X85-X86-X87-X88-X89-X90-X-1-X92-X93-X94-X95-X96-X97-X98-X99-X100-X101-X102-X103-X104-X105-X106-X107-X108-X109-X110-X111-X112-X113-X114-X115-X116-X117-X118-X119-X120-X121-X122-X123-X124-X125-X126-X127-X128-X129-X130-X131 wherein
| | |
|---|---|
| X1 = | amino group, amide, acetyl group, biotin group, marker, spacer, linker, GKK, SGKK or deletion, |
| X2 = | E* |
| X3 = | V# |
| X4 = | Q# |
| X5 = | L# |
| X6 = | V# |
| X7 = | E# |
| X8 = | S# |
| X9 = | G# |
| X10 = | G# |
| X11 = | G# |
| X12 = | L# |
| X13 = | V# |
| X14 = | Q# |
| X15 = | P* |
| X16 = | G* |
| X17 = | G# |
| X18 = | S# |
| X19 = | L# |
| X20 = | R# |
| X21 = | L# |
| X22 = | S# |
| X23 = | C# |
| X24 = | T# |
| X25 = | A# |
| X26 = | S# |
| X27 = | G# |
| X28 = | Y* |
| X29 = | T* |
| X30 = | F* |
| X31 = | S* |
| X32 = | H#, R#, K# |
| X33 = | R#, H#, K# |
| X34 = | Y#, W#, F#, I#, L#, V#, T#, S# |
| X35 = | H#, R#, K# |
| X36 = | R#, H#, K# |
| X37 = | W# |
| X38 = | F# |
| X39 = | R# |
| X40 = | Q# |
| X41 = | A# |
| X42 = | P* |
| X43 = | G* |
| X44 = | K* |
| X45 = | E# |
| X46 = | R# |
| X47 = | E# |
| X48 = | I# |
| X49 = | V# |
| X50 = | A# |
| X51 = | V# |
| X52 = | I# |
| X53 = | S#, T#, W#, V#, I#, L# |
| X54 = | Q, N |
| X55 = | S, T |
| X56 = | G, A |
| X57 = | M#, C#, V#, I#, L#, Y#, W#, F# |
| X58 = | R#, K#, H# |
| X59 = | T# |
| X60 = | Y# |
| X61 = | Y# |
| X62 = | A# |
| X63 = | D* |
| X64 = | S* |
| X65 = | V* |
| X66 = | K* |
| X67 = | G* |
| X68 = | R# |
| X69 = | F# |
| X70 = | T# |
| X71 = | I# |
| X72 = | S# |
| X73 = | R# |
| X74 = | D# |
| X75 = | N* |
| X76 = | A* |
| X77 = | K* |
| X78= | N* |
| X79 = | T# |
| X80 = | V# |
| X81 = | Y# |
| X82 = | L# |
| X83 = | Q# |
| X84 = | M# |
| X85 = | N# |
| X86 = | S* |
| X87 = | L* |
| X88 = | K*, R*, S*, T*, A*, C*, D*, E*, F*, G*, H*, I*, L*, M*, N*, P*, Q*, V*, W*, Y* |
| X89 = | P* |
| X90 = | E* |
| X91 = | D* |
| X92 = | T#, I#, Y#, F#, V#, C#, M#, W#, Q#, E#, S#, K#, R#, H#, L# |
| X93 = | A# |
| X94 = | M# |
| X95 = | Y# |
| X96= | Y# |
| X97 = | C# |
| X98 = | A# |
| X99 = | A# |
| X100 = | G#, A# |
| X101 = | T#, S#, F#, W#, I#, L#, V# |
| X102 = | R, K, H |
| X103 = | K, H, R |
| X104 = | N, Q |
| X105 = | V, I, L, M |
| X106 = | W, F, Y |
| X107 = | T, S |
| X108 = | R, K, H |
| X109 = | Q, N |
| X110 = | H, K, R |
| X111 = | P* |
| X112 = | F* |
| X113 = | D# |
| X114 = | Y# |
| X115 = | W# |
| X116 = | G# |
| X117 = | Q# |
| X118 = | G# |
| X119 = | T# |
| X120 = | Q# |
| X121 = | V# |
| X122 = | T# |
| X123 = | V# |
| X124 = | S# |
| X125 = | S* |
| X126 = | A* |
| X127 = | S* |
| X128 = | G* |
| X129 = | A* |
| X130 = | E* |
| X131 = | amino group, amide, acetyl group, biotin group, marker, spacer, linker, GKK, SGKK or deletion, |
wherein particularly binding or CDR-regions are underlined;
wherein particularly amino acids, which are marked with a star (*) can be modified to any one of amino acids selected from the group comprising A, C, D, E, F, G, H, I, K, L, M, N, P, Q, R, S, T, V, W, Y or can be deleted;
wherein amino acids, which are marked by a hash sign (#) are in particular beta-strands.

6. The peptide according to any of claims 1 to 5,
**characterized in that**
the linker and/or a spacer are selected from the group comprising:
α-aminocarboxylic acids as well as homo- and heterooligomers thereof, α,ω-aminocarboxylic acids and branched homo- or heterooligomers thereof, other aliphatic and/or aromatic amino acids as well as linear and branched homo- or heterooligomers; amino-oligoalkoxyalkylamines; maleinimidocarboxylic acid derivatives; oligomers of alkylamines; 4-alkylphenyl derivatives; 4-oligoalkoxyphenyl or 4-oligoalkoxyphenoxy derivatives; 4-oligoalkylmercaptophenyl or 4-oligoalkylmercaptophenoxy derivatives; 4-oligo-alkylaminophenyl or 4-oligoalkylaminophenoxy derivatives; (oligoalkylbenzyl)phenyl or 4-(oligoalkylbenzyl)phenoxy derivatives, as well as 4-(oligo-alkoxybenzyl)phenyl or 4-(oligoalkoxy-benzyl)phenoxy derivatives; trityl derivatives; benzyloxyaryl or benzyloxyalkyl derivatives; xanthen-3-yloxyalkyl derivatives; (4-alkylphenyl)- or ω-(4-alkylphenoxy)alkanoic acid derivatives; oligoalkylphenoxyalkyl or oligoalkoxyphenoxyalkyl derivatives; carbamate derivatives; amines; trialkylsilyl or dialkylalkoxysilyl derivatives; alkyl or aryl derivatives and/or combinations thereof.

7. The peptide according to any of claims 1 to 6 for use as a medical active substance.

8. The peptide according to any of claims 1 to 7,
**characterized in that**
the peptide is bound by amyloid fibrillar and/or non-fibrillar polypeptides of patients suffering from amyloid disease.

9. The peptide according to any of claims 1 to 8,
**characterized in that**
the peptide is immobilized and/or fixed to magnetic, paramagnetic and/or non magnetic nanoparticles.

10. The peptide according to one of the preceding claims,
**characterized in that**
the peptide is bound to a solid phase.

11. An isolated nucleic acid molecule selected from the group comprising:
a) a nucleic acid molecule comprising a nucleotide sequence which encodes at least one peptide selected from the group consisting of peptides according to claims 1 to 5, particularly according to SEQ ID No. 1, preferably according to SEQ ID No. 2, 3 and/or 4;
b) a nucleic acid molecule which is complementary to a nucleotide sequence in accordance with a);
c) a nucleic acid molecule which undergoes hybridization with a nucleotide sequence according to a) or b) under stringent conditions;
d) a nucleic acid molecule comprising a nucleotide sequence having sufficient homology to be functionally analogous/equivalent to a nucleotide sequence according to a), b) or c);
e) a nucleic acid molecule which, as a consequence of the genetic code, is degenerated into a nucleotide sequence according to a) through d); and
f) a nucleic acid molecule according to a nucleotide sequence of a) through e) which is modified by deletions, additions, substitutions, translocations, inversions and/or insertions and functionally analogous/equivalent to a nucleotide sequence according to a) through e).

12. The nucleic acid molecule according to claim 11,
**characterized in that**
the nucleotide sequence specified under d) has at least 40% homology to any of the nucleotide sequences specified under a) through c).

13. The nucleic acid molecule according to claim 11,
**characterized in that**
the nucleotide sequence specified under d) has at least 60%, preferably 70%, more preferably 80%, especially preferably 90% homology to any of the nucleotide sequences specified under a) through c).

14. The nucleic acid molecule according to any of claims 11 to 13,
**characterized in that**
it is a genomic DNA, a cDNA and/or an RNA.

15. A vector comprising a nucleic acid molecule according to any of claims 11 to 14.

16. A host cell comprising the vector according to claim 15.

17. A peptide, said peptide being encoded by a nucleic acid molecule according to any of claims 11 to 14.

18. A Recognition-molecule comprising the peptide according to claims 1 to 5.

19. The Recognition-molecule according to claim 18,
**characterized in that**
the recognition-molecule is an antibody-fragment of an antibody or an antibody.

20. The Recognition-molecule according to claims 18 or 19,
**characterized in that**
the antibody or the fragment thereof is a chimeric antibody or a fragment thereof.

21. The Recognition-molecule according to any of claims 18 to 20,
**characterized in that**
the chimeric antibody or the fragment thereof is a partially humanized antibody or a fragment thereof.

22. The Recognition-molecule according to any of claims 18 to 21,
**characterized in that**
the fragment of the antibody is a variable fragment of a single-chain of an antibody.

23. The Recognition-molecule according to any of claims 18 to 22,
**characterized in that**
the single-chain is a heavy chain variable domain.

24. Solid phases for affinity chromatography or solid-phase extraction consisting of organic, inorganic, synthetic polymers or of mixed polymers, preferably cross-linked agarose, cellulose, silica gel, polyamide and polyvinyl alcohols, which are optionally chemically activated, with peptides or a recognition-molecule according to at least one of claims 1 to 10 or 17 to 23 immobilized on the surface of the solid phase.

25. The solid phases according to claim 24, wherein the peptides are bound to the solid support phase covalently or by adsorption.

26. The solid phases according to claim 24 or 25,
wherein the peptides are covalently bound to the solid phase on any of positions X1 to X135.

27. The solid phases according to any of claims 24 to 26, wherein the peptides are distanced from the support surface by linkers/spacers.

28. A device for removing amyloid fibrillar and/or non-fibrillar polypeptides from samples on solid phases, wherein the device contains a solid phase according to any of claims 24 to 25, and means for the entry of samples are provided.

29. A pharmaceutical composition comprising a nucleic acid molecule according to any of claims 11 to 14, a vector according to claim 15, a host cell according to claim 16, a peptide according to any of claims 1 to 10, a recognition-molecule according to claims 18 to 23 and/or a solid phase according to claims 24 to 27, optionally together with a pharmaceutically tolerable carrier.

30. The pharmaceutical agent according to the preceding claim,
**characterized in that**
the carrier is selected from the group comprising fillers, disintegrants, binders, humectants, extenders, dissolution retarders, absorption enhancers, wetting agents, adsorbents and/or lubricants.

31. The pharmaceutical agent according to any of the preceding claims,
**characterized in that**
said agent is a capsule, a tablet, a coated tablet, a suppository, an ointment, a cream, an injection solution and/or an infusion solution.

32. The pharmaceutical agent according to any of the preceding claims,
**characterized in that**
said agent is an oral, vaginal, rectal, nasal, topical, subcutaneous, intravenous, intramuscular, intraperitoneal agent, suppository, pad and/or foam.

33. A kit comprising a nucleic acid molecule according to any of claims 11 to 14, a vector according to claim 15, a host cell according to claim 16, a peptide according to any of claims 1 to 10, a recognition-molecule according to claims 18 to 23, a solid phase according to claims 24 to 27 and/or a pharmaceutical composition according to claim 29, optionally together with instructions for combining the contents of the kit and/or providing a formulation.

34. Kit according to claim 33,
**characterized in that**,
the antibody comprises phosphatase moiety which allows in a preferred embodiment a direct detection of amyloidoses.

35. Use of the kit according to claims 33 or 34 for diagnosis of amyloid diseases.

36. Use of the kit according to claims 33 or 34 for conformation-sensitive detection of amyloid.

37. An apparatus for chromatography, comprising the recognition-molecule according to any of claims 18 to 23.

38. The apparatus according to claim 37,
**characterized in that**
the recognition-molecules are bound to a solid phase according to claims 24 to 27.

39. Use of a nucleic acid molecule according to any of claims 11 to 14, a vector according to claim 15, a host cell according to claim 16, a peptide according to any of claims 1 to 10, a recognition-molecule according to claims 18 to 23, a solid phase according to claims 24 to 27, a pharmaceutical composition according to claim 29 to 32, a kit according to claim 33 or 34, an apparatus according to claim 37 or 38 in the prophylaxis, diagnosis, therapy, follow-up and/or aftercare of amyloid diseases.

40. The use according to claim 39, wherein the peptide, the nucleic acid molecule according to any of claims 11 to 14, the vector according to claim 15, the host cell according to claim 16, the recognition-molecule according to claims 18 to 23, the solid phase according to claims 24 to 27, the pharmaceutical composition according to claim 29 to 32, the kit according to claim 33 or 34, the apparatus according to claim 37 or 38 modify the aggregation of amyloid proteins, in particular by promoting a non-fibrillar aggregation.

41. The use according to claim 39 or 40,
**characterized in that**
the amyloid diseases are selected from the group comprising
- Systemic amyloidosis
• Primary amyloidosis
■ Mutations in lysozyme, transthyretin, apolipoprotein B, fibrinogen
■ AL amyloidosis
• Secondary amyloidosis
■ AA amyloidosis
■ Gelsolin amyloidosis
• Familial or Hereditary amyloidosis
■ Caused by mutations in the transthyretin protein or caused by apolipoprotein A1, gelsolin, fibrinogen, and lysozyme mutations
■ Appalachian type amyloidosis
■ Shar Pei fever for amyloidosis in Shar Peis
- Organ-specific amyloidosis
• Familial or Hereditary amyloidosis
• Diabetes mellitus type 2
• Neurology
■ Alzheimer's disease (Aβ 39-42)
■ Parkinson's disease (alpha-synuclein)
■ Huntington's disease (huntingtin)
■ Transmissible spongiform encephalopathies (prion protein, PrP)
- Creutzfeldt-Jakob disease (PrP in cerebrum)
- Kuru (diffuse PrP deposits in brain)
- Fatal Familial Insomnia (PrP in thalamus)
- Bovine spongiform encephalopathy (PrP in cerebrum of cows)
■ Congophilic angiopathy (Amyloid beta)
• Cardiac amyloidosis
■ congestive heart failure
• Inclusion body myositis
- Iatrogenic conditions
• insulin amyloidosis.

42. A method for the treatment of said amyloid diseases by binding and/or removal of the amyloid fibrillar polypeptides or the non-fibrillar polypeptides by means of the recognition-molecules according to any of claims 18 to 23.


SEQUENCE LISTING

<110>  Leibniz-Insitut für Naturstoff-Forschung und
       Infektionsbiologie Hans-Knöll-Institut
       Leibniz-Insitut für Altersforschung Fritz-Lippmann-Institut
        e.V.

<120>  Anti-amyloid antibodies and their use in the therapy of amyloid
       diseases

<130>  XII 397-07

<160>  4

<170>  PatentIn version 3.3

<210>  1
<211>  129
<212>  PRT
<213>  Artificial

<220>
<223>  Artificial sequence used as a binding protein for amyloid
       structures

<400>  1

Glu Val Gln Leu Val Glu Ser Gly Gly Gly Leu Val Gln Pro Gly Gly
1               5                   10                  15

Ser Leu Arg Leu Ser Cys Thr Ala Ser Gly Tyr Thr Phe Ser His Arg
            20                  25                  30

Tyr His Arg Trp Phe Arg Gln Ala Pro Gly Lys Glu Arg Glu Ile Val
        35                  40                  45

Ala Val Ile Ser Gln Ser Gly Met Arg Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Met Tyr Tyr Cys
                85                  90                  95

Ala Ala Gly Thr Arg Lys Asn Val Trp Thr Arg Gln His Pro Phe Asp
            100                 105                 110

Tyr Trp Gly Gln Gly Thr Gln Val Thr Val Ser Ser Ala Ser Gly Ala
        115                 120                 125

Glu


<210>  2
<211>  5
<212>  PRT
<213>  Artificial

<220>
<223>  Artificial sequence used as a binding protein for amyloid
      structures

<400>  2

His Arg Tyr His Arg
1               5


<210>  3
<211>  6
<212>  PRT
<213>  Artificial

<220>
<223>  Artificial sequence used as a binding protein for amyloid
      structures

<400>  3

Ser Gln Ser Gly Met Arg
1               5


<210>  4
<211>  12
<212>  PRT
<213>  Artificial

<220>
<223>  Artificial sequence used as a binding protein for amyloid
      structures

<400>  4

Gly Thr Arg Lys Asn Val Trp Thr Arg Gln His Pro
1               5                   10